# EUROPÄISCHE PATENTANMELDUNG

(11) **EP 2 711 198 A1**
(43) Veröffentlichungstag der Anmeldung: **26.03.2014**
(21) Anmeldenummer: 12185724.7
(22) Anmeldetag: 24.09.2012
(51) Int. Cl.: B60C 1/00, C07C 323/62, C08K 5/36, C08K 5/54, C08L 21/00

(54) **Polysulfid-Additive, Verfahren zu deren Herstellung und deren Verwendung in Kautschukmischungen**

(71) Anmelder: LANXESS Deutschland GmbH, 50569 Köln (DE)
(72) Erfinder: Feldhues, Ulrich, 51465 Bergisch Gladbach (DE); Unterberg, Heinz, 41540 Dormagen (DE); Wiedemeier-Jarad, Melanie, 41540 Dormagen (DE)

(57) **Zusammenfassung**

Die vorliegende Erfindung betrifft Polysulfide der Formel (I), 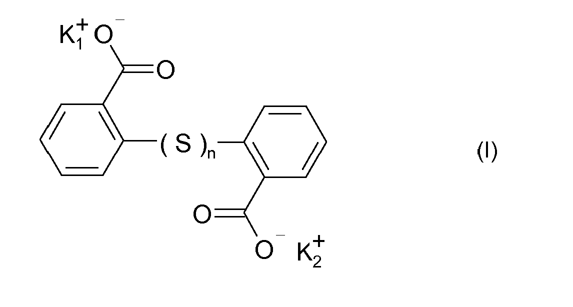 wobei die Kationen K₁⁺ und K₂⁺ unabhängig voneinander für beliebige einwertige oder für den Anteil eines beliebigen mehrwertigen Kations stehen, der einer positiven Ladung von eins entspricht und n für 2, 3, 4, 5, 6 steht, welche eine geringe Restacidität aufweisen, sowie Verfahren zur Herstellung dieser Polysulfid-Mischungen, die Verwendung der Polysulfide der Formel (I) in Kautschukmischungen, die entsprechenden Kautschukmischungen, daraus hergestellte Kautschukvulkanisate und deren Verwendung.

## Beschreibung

Kieselsäurehaltige Kautschukmischungen sind wichtige Ausgangsmaterialien, beispielsweise für die Herstellung von Reifen mit verringertem Rollwiderstand. Diese leisten beim Abrollen weniger Verformungsarbeit (als Reifen, welche allein Ruß als Füllstoff enthalten) und senken deshalb den Kraftstoffverbrauch. Durch die in verschiedenen Staaten beschlossene Kennzeichnungspflicht des Rollwiderstands für Reifen besteht ein großes Interesse daran, diesen Widerstand weiter zu senken.

Zur Verbesserung der physikalischen Eigenschaften der Vulkanisate werden, wie beispielsweise in DE 2 255 577 A erwähnt, polysulfidische Silane als Verstärkungsadditive eingesetzt. Das Eigenschaftsprofil der so hergestellten Kautschukvulkanisate ist noch nicht optimal. Neben einer Verbesserung des Rollwiderstands ist insbesondere eine niedrige Fließviskosität (Mooney Viskosität ML 1+4/100°C) der Kautschukmischung erwünscht, was die Verarbeitbarkeit begünstigt. Hierfür wurden weitere Zusatzstoffe vorgeschlagen, wie Fettsäureester, Fettsäuresalze oder Mineralöle, welche zwar die Fließfähigkeit erhöhen, gleichzeitig aber die Spannungswerte bei größerer Dehnung (z.B. 100% bis 300%) oder auch die Härte der Vulkanisate vermindern, so dass die verstärkende Wirkung des Füllstoffes nicht mehr voll zum Tragen kommt. Eine zu geringe Härte oder Steifigkeit des Vulkanisats resultiert aber in unbefriedigendes Fahrverhalten des Reifens besonders in Kurven. Zur Erhöhung der Härte des Vulkanisats kann der Anteil an verstärkendem Füllstoff erhöht, oder der Anteil an Weichmacheröl verringert werden, wobei jede dieser beiden Maßnahmen den Nachteil der höheren Mischungsviskosität bei der Verarbeitung bedingt.

In EP 0 489 313 werden Glykolfunktionen enthaltende Additive mit guten mechanischen Eigenschaften und verbessertem Hystereseverhalten beschrieben. Im Vergleich mit dem Bis-[-3-(triethoxysilyl)-propyl]-tetrasulfid gemäß DE-OS 2 255 577 zeigen die Beispiele aber keine Verbesserung im Rollwiderstand (tan δ bei 60°C).

In EP 1 000 968 konnte eine Verbesserung der physikalischen Eigenschaften durch Verwendung eines polysulfidischen Silans in Kombination mit einem speziellen Reversionsschutzmittel in SBR erzielt werden, wobei aber weder bei der Mischungsviskosität noch beim Rollwiderstand (tan δ bei 60°C) eine signifikante Änderung gegenüber dem Stand der Technik erzielt wurde.

In der bis dato unveröffentlichten Anmeldung EP 11164319 ist die Verwendung von mittels Dischwefeldichlorid hergestellten Dibenzoesäuretetrasulfid zusammen mit Bis-[-3-(triethoxysilyl)-propyl]-tetrasulfid in kieselsäurehaltigen Kautschukmischungen beschrieben. Gegenüber einer Referenzmischung ohne Dibenzoesäuretetrasulfid war die Verbesserung der Mooney Viskosität nur marginal, zudem wurde die Zugfestigkeit etwas beeinträchtigt und der Rollwiderstand sowie der Abrieb deutlich verschlechtert.

Die Herstellung von 2,2'-Tetrathiobisbenzoesäure ist bislang nur in der US 1769423 aus dem Jahr 1930 offenbart. Dabei wurde offensichtlich ohne Inertgas gearbeitet, wodurch ein Teil der sehr oxidationsempfindlichen Mercaptobenzoesäure zu 2,2'-Dithiobisbenzoesäure reagiert und für die Reaktion mit S₂Cl₂ dann nicht mehr zur Verfügung steht. Zudem wird S₂Cl₂ in ca. 15%igem Überschuss eingesetzt, wodurch das Produkt einen signifikanten Anteil an Chlor-haltigen Nebenkomponenten und eine hohe Acidität aufweist. Die beschriebene Umkristallisation aus Amylalkohol sowie anderen polaren Lösungsmitteln zeigte sich bei eigenen Versuchen als zur Aufarbeitung der Polysulfide ungeeignet und führte zur Zersetzung des Tetrasulfids, zur Veresterung der Carbonsäuregruppe bzw. zum Austausch aktiver Schwefelatome zwischen den Polysulfiden und somit zur Erhöhung der Anteile an 2,2'-Dithiobisbenzoesäure, 2,2'-Trithiobisbenzoesäure, 2,2'-Pentathiobisbenzoesäure und 2,2'-Hexathiobisbenzoesäure, d. h. zur Verbreiterung der Verteilungskurve des Produktgemisches. Für die Verwendung in Kautschuk ist dies sehr nachteilig, da sich durch Bildung deutlich schwächer wirkender Disulfide und Trisulfide eine Reduzierung der Wirkung der Polysulfide ergibt.

Als aktive Schwefelatome sind dabei Schwefelatome in Polysulfiden zu verstehen, welche nur Schwefelatome als Bindungspartner haben. Der Austausch von aktiven Schwefelatomen führt dabei nicht zu einer Änderung der elementaren Zusammensetzung einer Polysulfid-Mischung, lässt sich also über Elementaranalyse nicht nachweisen. Der Nachweis von Polysulfidverteilungen gelingt jedoch durch HPLC, wie von Douglas E. Doster und Melodee Zentner im Journal of Chromatography, 461 (1989) 293-303 und Arisawa Mieko et. al in Tetrahedron Letters, 2005, Volume 46, Ausgabe 28, 4797-4800 beschrieben. Dabei wurde deutlich, dass die bestimmten Polysulfide als Polysulfid-Mischungen mit einer sehr breiten Verteilung an Molekülen mit unterschiedlich vielen Schwefelatomen (S2, S3, S4, S5, S6) vorliegen.

Aufgabe der vorliegenden Erfindung ist die Bereitstellung neuer Kautschukadditive, Verfahren zu deren Herstellung und neuer Kautschukmischungen, welche bei sehr guter Fließfähigkeit der Kautschukmischungen in Vulkanisate mit vermindertem Rollwiderstand überführt werden können, bei denen aber zugleich die ebenfalls wichtigen Parameter Shore A Härte, Modul 300, Bruchdehnung, Zugfestigkeit und Abrieb keine wesentliche Beeinträchtigung zeigen.

Es wurde nun gefunden, dass bei der Umsetzung von 2-Thiobenzoesäure mit S₂Cl₂ erhaltene Polysulfide eine durch einfaches Mischen mit Wasser nicht feststellbare Restacidität enthalten. So zeigte eine Mischung von 10g des gemäß EP 11164319 erhaltenen 2,2'-Tetrathiobisbenzoesäureprodukts mit 100ml Wasser bei 25 °C einen pH von 4,1 und eine Leitfähigkeit von 0,02 mS/cm. Überraschenderweise betrug der pH der wässrigen Phase nach 30-minütigem Erhitzen der Mischung zum Rückfluss und anschließendem Abkühlen auf 25°C 1,2 und die Leitfähigkeit 14mS/cm.

Überraschenderweise zeigte sich, dass durch Nachbehandlung der bei der Umsetzung von 2-Thiobenzoesäure mit S₂Cl₂ erhaltenen Polysulfide in einem unpolaren organischen Lösungsmittel, in dem die Polysulfide vorzugsweise nicht oder nur unwesentlich löslich sind, ausgewählt aus unter Reaktionsbedingungen flüssigen cyclischen und/oder acyclischen Kohlenwasserstoffen, aromatischen Kohlenwasserstoffen, aliphatischen und/oder aromatischen Halogenkohlenwasserstoffen, Ethern und Estern, insbesondere Toluol, und Wasser bei erhöhter Temperatur, vorzugsweise bei einer Temperatur > 60°C, besonders bevorzugt bei einer Temperatur im Bereich von 80 - 120°C, es nun erstmals möglich war, solche Polysulfide derart herzustellen, dass sie eine deutlich verminderte Restacidität und Chlor-Gehalt aufweisen, ohne dass es durch die Nachbehandlung zu einer signifikanten Verbreiterung der Schwefelverteilung der Polysulfide oder zu deren Zersetzung kommt, so dass die erhaltenen Polysulfide bei Verwendung als Kautschukadditiv die oben geforderten Eigenschaftsverbesserungen bewirken.

Die Mischungen von 10g der erfindungsgemäßen Polysulfide und 100ml Wasser zeigen nach 30-minütigem Erhitzen zum Rückfluss bei Normaldruck und anschließendem Abkühlen auf 25°C einen pH > 2, bevorzugt von 2,5 - 8, besonders bevorzugt von 3 - 7, ganz besonders bevorzugt von 3,4 - 6,2. Die abgekühlten Mischungen weisen typischerweise eine Leitfähigkeit < 5 mS/cm, besonders bevorzugt < 1 mS/cm auf. Bei diesen Messungen soll die Gesamtdauer der Abkühlphase zusammen mit der Verweildauer bis zur Bestimmung des pH-Werts und des Leitfähigkeitswerts soll dabei eine Stunde nicht überschreiten.

Zudem zeigte sich, dass durch die obige Nachbehandlung der Polysulfide deren Chlorgehalt von deutlich über 1 % stark reduziert wird. Bevorzugt weisen die erfindungsgemäßen Polysulfide einen Chlor-Gehalt von < 1%, bevorzugt < 0,3%, besonders bevorzugt < 0,1%, und ganz besonders bevorzugt < 0,03% auf.

Die erfindungsgemäßen Polysulfide bestehen aus Verbindungen der Formel (I), 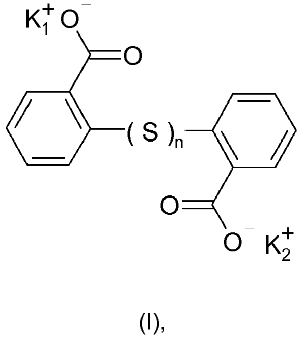 wobei
- K₁⁺ und K₂⁺: unabhängig voneinander für ein einwertiges Kation oder für den Anteil eines mehrwertigen Kations stehen, der einer positiven Ladung von eins entspricht, und
- n: für 2, 3, 4, 5 und/oder 6 steht.

In einer bevorzugten Ausführungsform stehen K₁⁺ und K₂⁺ unabhängig voneinander für H⁺, ein Alkalimetallkation, insbesondere Li⁺, Na⁺, K⁺, ½ Erdalkalimetallkation, insbesondere ½ Mg²⁺, ½Ca²⁺, für 1/3 Al³⁺, für den Anteil eines Seltenerdmetallkations, der einer positiven Ladung von eins entspricht, oder für ½Zn²⁺.

Ganz besonders bevorzugt stehen K₁⁺ und K₂⁺ unabhängig voneinander für H⁺ oder für ½ Zn-Kation, insbesondere für ½Zn-Kation.

Polysulfidverbindung(en) der Formel (I) mit n = 2, 3, 4, 5 und/oder 6 bei denen K₁⁺ und/oder K₂⁺ für ½ Zn²⁺ stehen, sind ein bevorzugter Gegenstand der vorliegenden Erfindung. Ganz besonders bevorzugt sind Polysulfidverbindung(en) der Formel (1) mit n = 4 bei denen K₁⁺ und/oder K₂⁺ für ½ Zn²⁺ stehen, insbesondere solche bei denen K₁⁺ und K₂⁺ für ½ Zn²⁺ stehen.

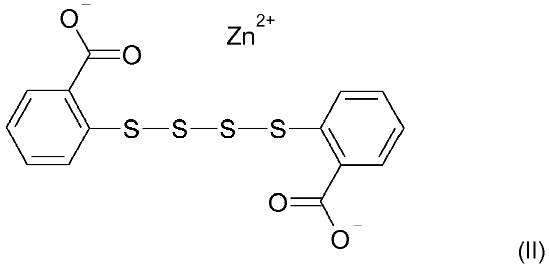

Die erfindungsgemäßen Polysulfide bestehen typischerweise aus mehreren Verbindungen der Formel (I), wobei bezogen auf die Verbindungen der Formel (I) der aufsummierte Anteil der Verbindungen der Formel (I) mit n = 3, n = 4, n = 5 und n = 6 wenigstens 80 %, vorzugsweise mindestens 90 % besonders bevorzugt mindestens 95% und ganz besonders bevorzugt mindestens 98 % beträgt. Die Prozentangaben der Anteile in dieser Erfindung zu den Verbindungen der Formel (I) ergeben sich dabei immer direkt aus den Flächenprozentangaben der HPLC-Messung wie nachfolgend in Rahmen der Beispiele angegeben.

In einer besonders bevorzugten Ausführungsform beträgt der Anteil der Verbindung der Formel (I) mit n = 4 bezogen auf die Verbindungen der Formel (I) mehr als 80%, besonders bevorzugt mehr als 90%, ganz besonders bevorzugt mehr als 95%, und am meisten bevorzugt 96 - 99%.

Bevorzugt beträgt der Anteil der Verbindung der Formel (I) mit n = 2 bezogen auf die Verbindungen der Formel (I) weniger als 10%, besonders bevorzugt weniger als 5%, ganz besonders bevorzugt weniger als 2% und am meisten bevorzugt weniger als 1 %.

Erfindungsgemäß bevorzugte Polysulfide auf Basis von Verbindungen der Formel (I) weisen einen Schwefelgehalt nach Elementaranalyse zwischen 28 - 40%, insbesondere zwischen 32 - 36% auf.

Typischerweise weisen die erfindungsgemäßen Polysulfide als Mischung der Verbindungen der Formel (I) im Durchschnitt 3,5 - 4,5, besonders bevorzugt 3,7 - 4,3, ganz besonders bevorzugt 3,8 - 4,2 und am meisten bevorzugt 3,9 - 4,1 Schwefelatome pro Molekül der Formel (I) auf.

Bevorzugte Polysulfide auf Basis von Verbindungen der Formel (I) enthalten vorzugsweise weniger als 10 Gew.-%, besonders bevorzugt weniger als 3 Gew.-%, ganz besonders bevorzugt weniger als 1 Gew.-% an Nebenprodukten oder Beimischungen, also an Verbindungen, die nicht der Formel (I) entsprechen auf. Insbesondere beträgt der Gehalt an elementarem Schwefel und/oder Schwefelspendern bezogen auf Verbindungen der Formel (I) weniger als 2%, besonders bevorzugt weniger als 1%, ganz besonders bevorzugt weniger als 0,3%, meist bevorzugt weniger als 0,1% und der Gehalt an Beschleunigern der Mercapto- bzw. Sulfenamid-Gruppe bezogen auf Verbindungen der Formel (I) weniger als 2%, besonders bevorzugt weniger als 1%, ganz besonders bevorzugt weniger als 0,3% und meist bevorzugt weniger als 0,1%.

Die vorliegende Erfindung betrifft auch ein Verfahren zur Herstellung der erfindungsgemäßen Polysulfide der Formel (I) durch Umsetzung von 2-Mercaptobenzoesäure mit S₂Cl₂ zu Verbindung(en) der Formel (I), wobei K₁⁺ und K₂⁺ für H⁺ stehen und n für 2, 3, 4, 5, oder 6, vorzugsweise für 4 steht. Bevorzugt wird die Reaktion in einem inerten Reaktionsmedium unter Schutzgasatmosphäre bei Temperaturen zwischen 0°C und 60°C, insbesondere zwischen 15°C und 35°C durchgeführt. Bei zu niedrigen Temperaturen reagiert S₂Cl₂ ggf. nicht sofort komplett ab, was zu Nebenprodukten und zu einem Gefährdungspotential durch den Aufbau einer erhöhten S₂Cl₂ - Konzentration im Reaktionsansatz führen kann. Zu hohe Temperaturen sind aus Gründen der Produktqualität und Arbeitssicherheit ebenfalls zu vermeiden.

Inerte Reaktionsmedien sind Reaktionsmedien, die unter den Reaktionsbedingungen nicht oder nur unwesentlich mit den Reaktionskomponenten reagieren, insbesondere unter Reaktionsbedingungen flüssige aliphatische cyclische und/oder acyclische Kohlenwasserstoffe, aromatische Kohlenwasserstoffe, aliphatische und/oder aromatische Halogenkohlenwasserstoffe, Ether und Ester. Besonders bevorzugtes Reaktionsmedium ist Toluol. Üblicherweise werden wasserfreie oder getrocknete Reaktionsmedien verwendet, um Nebenreaktionen mit S₂Cl₂ zu vermeiden. Es können auch Gemische als Reaktionsmedien eingesetzt werden. Vorzugsweise werden Reaktionsmedien und Reaktionsbedingungen verwendet, unter denen das Reaktionsprodukt nicht oder nur unwesentlich gelöst wird.

Typischerweise wird das Reaktionsmedium unter Schutzgas vorgelegt, Mercaptobenzoesäure eingetragen und dann S₂Cl₂ unter Kühlung des Reaktionsansatzes zugegeben. Mercaptobenzoesäure und S₂Cl₂ können auch gleichzeitig eingetragen werden. Mercaptobenzoesäure kann auch vorgelegt werden oder gleichzeitig mit einem Reaktionsmedium eingetragen werden. Mercaptobenzoesäure und S₂Cl₂ werden bevorzugt in Verhältnissen eingesetzt, die sich aus der Stöchiometrie ergeben, wobei die Abweichungen < +/- 5%, bevorzugt < +/- 2%, besonders bevorzugt < +/- 1% betragen. Besonders bevorzugt wird ein Überschuss an S₂Cl₂ vermieden.

Als Inertgase werden bei der Synthese der Polysulfide der Formel (I) vorzugsweise Edelgase oder Stickstoff, insbesondere Stickstoff verwendet. In einer bevorzugten Ausführungsform wird das entstehende HCl-Gas bereits während der Reaktion ganz oder anteilig aus dem Ansatz entfernt, insbesondere durch Durchleiten von Inertgas, insbesondere Stickstoff, durch den Reaktionsansatz und/oder durch Anlegen von Vakuum.

Das vorliegende Verfahren umfasst weiterhin das Inkontaktbringen der erhaltenen Polysulfide der Formel (I) mit Wasser oder einem Gemisch von Wasser und einem inerten organischen Medium, in dem die Polysulfide vorzugsweise nicht oder nur unwesentlich löslich sind, insbesondere in unter Reaktionsbedingungen flüssigen cyclischen und/oder acyclischen Kohlenwasserstoffen, aromatischen Kohlenwasserstoffen, aliphatischen und/oder aromatischen Halogenkohlenwasserstoffen, Ethern und Estern, besonders bevorzugt Toluol, und Erwärmung auf eine Temperatur > 60°C, bevorzugt auf 80°C - 120°C. Die Dauer der Erwärmung beträgt vorzugsweise mehr als 30 Minuten, besonders bevorzugt 1h - 10h, ganz besonders bevorzugt 2h - 6h. Vorzugsweise wird die Umsetzung mit S₂Cl₂ mit Mercaptobenzoesäure und die nachgelagerte Behandlung mit Wasser ohne Zwischenisolierung insbesondere in einem Eintopfverfahren durchgeführt.

In einer alternativen Ausführungsform werden, an Stelle der, oder in Anschluss an die obige Behandlung mit Wasser, die Polysulfide der Formel (I), bei denen K₁⁺ und/oder K₂⁺ für H⁺ stehen mit Salzen von Metallen enthaltend Kationen K₁⁺ und/oder K₂⁺ in Kontakt gebracht und vorzugsweise in wässriger Dispersion auf eine Temperatur > 60°C, bevorzugt auf 80°C - 120°C erwärmt, wodurch erfindungsgemäße Polysulfide der Formel (I), in denen wenigstens eines der Kationen K₁⁺ und/oder K₂⁺ für ein anderes Kation als H⁺ stehen, erhalten werden. Die Dauer der Erwärmung beträgt vorzugsweise mehr als 30 Minuten, besonders bevorzugt 1h - 10h, ganz besonders bevorzugt 2h - 6h. Der pH-Wert liegt bei der Herstellung der Polysulfide der Formel (I) , in denen wenigstens eines der Kationen K₁⁺ und/oder K₂⁺ für ein anderes Kation als H⁺ stehen, vorzugsweise im Bereich von 2 - 8, insbesondere im Bereich von 3 - 7. Als Metallsalzlösungen werden vorzugsweise Sulfate, Hydrogensulfate, Phosphate, Hydrogenphosphate, Dihydrogenphosphate, Carbonate, Hydrogencarbonate, Hydroxide, Nitrate, Chloride und Acetate, insbesondere Sulfate eingesetzt. Alkalihydroxide, insbesondere Alkalihydroxyd-Lösungen werden bevorzugt als Hilfsbasen eingesetzt, um einen optimalen Reaktions-pH-Bereich einzustellen. Die durch die Umsetzung mit den Metallsalzen erhaltenen Verbindungen der Formel (I), insbesondere Verbindungen der Formel (I), bei denen K₁⁺ und/oder K₂⁺ für ½ Zn²⁺ stehen, weisen nicht nur den nach 30 min Erhitzen von 10g Polysulfiden in 100ml Wasser zum Rückfluss und anschließendem Abkühlen auf 25°C geforderten pH auf, sondern zeigen überraschenderweise auch eine deutliche Verbesserung des Eigenschaftsprofils von vulkanisierten Kautschukmischungen wie die erfindungsgemäßen Verbindungen der Formel (I) bei denen K₁⁺ und/oder K₂⁺ für H⁺ stehen.

Das erfindungsgemäße Verfahren kann in Batch-Fahrweise, in kontinuierlicher Fahrweise oder Kaskadenfahrweise durchgeführt werden.

Durch das erfindungsgemäße Verfahren werden Verbindungen der Formel (I) zugänglich, welche bei Mischung von 10g der Verbindungen der Formel (I) mit 100ml Wasser nach 30-minütigem Erhitzen zum Rückfluss und anschließendem Abkühlen auf 25°C einen pH > 2, bevorzugt von 2,5 bis 8, besonders bevorzugt von 3 - 7, ganz besonders von 3,4 - 6,2 aufweisen. Die abgekühlten Mischungen weisen typischerweise eine Leitfähigkeit < 5 mS/cm, besonders bevorzugt < 1 auf.

Der Chlor-Gehalt der erfindungsgemäßen Polysulfide beträgt < 1%, bevorzugt < 0,3%, besonders bevorzugt < 0,1%, und ganz besonders bevorzugt < 0,03% auf.

Das erfindungsgemäße Verfahren ermöglicht es zudem Polysulfide auf Basis von Verbindungen der Formel (I) mit einer sehr geringen Schwefelverteilungsbreite, d.h. einem sehr hohen Anteil an Verbindungen der Formel (I) mit n = 4 und einem sehr niedrigen Anteil an Verbindungen der Formel (I) mit n = 2 insbesondere mit einem sehr niedrigen Anteil an Verbindungen der Formel (I) mit n = 2, 3, 5, und 6, herzustellen.

Überraschend konnte auch die bei diesen Verbindungen übliche starke Geruchsbelästigung ausgeräumt werden, da die erhaltenen Polysulfide nur einen schwachen Eigengeruch aufweisen.

Vorzugsweise werden die erfindungsgemäßen Polysulfide der Formel (I) nach der Herstellung bei Temperaturen zwischen 0 - 35°C gelagert.

Überraschend wurde nun gefunden, dass die erfindungsgemäßen Polysulfide der Formel (I) die Fließfähigkeit von Kautschukmischungen verbessern und dabei Vulkanisate mit einem guten Eigenschaftsprofil und insbesondere geringem Rollwiderstand erhalten werden.

Ein weiterer Gegenstand der vorliegenden Erfindung sind Polysulfide der Formel (I) erhältlich durch Umsetzung von 2-Mercaptobenzoesäure mit S₂Cl₂ zu Verbindung(en) der Formel (I), wobei K₁⁺ und K₂⁺ für H⁺ stehen und n für 2, 3, 4, 5, oder 6, vorzugsweise für 4 steht, vorzugsweise durch Reaktion in einem inerten Reaktionsmedium unter Schutzgasatmosphäre bei Temperaturen zwischen 0°C und 60°C, insbesondere zwischen 15°C und 35°C, und Nachbehandlung durch Suspension in einem inerten organischen Medium, in dem die Polysulfide vorzugsweise nicht oder nur unwesentlich löslich sind, ausgewählt aus unter Reaktionsbedingungen flüssige cyclische und/oder acyclische Kohlenwasserstoffe, aromatische Kohlenwasserstoffe, aliphatische und/oder aromatische Halogenkohlenwasserstoffe, Ether und Ester, insbesondere Toluol und Inkontaktbringen mit Wasser bei Temperaturen > 60°C, insbesondere 80 - 120°C. Ganz besonders bevorzugt wird die Reaktionssuspension nach der Zuführung von Wasser zum Rückfluss erhitzt und/oder ein Teil des Reaktionsmediums insbesondere als Azeotrop mit Wasser abdestilliert. Ein weiterer Gegenstand der Erfindung sind daher Kautschukmischungen, enthaltend jeweils mindestens einen Kautschuk und die erfindungsgemäßen Polysulfide der Formel (I).

Gegenstand der Erfindung sind insbesondere Kautschukmischungen, enthaltend jeweils mindestens einen Kautschuk, ein schwefelhaltiges Alkoxysilan, einen kieselsäurebasierenden Füllstoff und die erfindungsgemäßen Verbindungen der Formel (I).

Die erfindungsgemäßen Polysulfide der Formel (I) können auch teilweise oder vollständig auf inerten, organischen oder anorganischen Trägern absorbiert eingesetzt werden. Bevorzugte Trägermaterialien sind Kieselsäure, natürliche und synthetische Silikate, Aluminiumoxid und/oder Russe.

Der Gesamtgehalt der erfindungsgemäßen Polysulfide der Formel (I) in bevorzugten Kautschukmischungen beträgt 0,1 bis 15 phr, besonders bevorzugt 0,3 bis 7 phr, ganz besonders bevorzugt 0,5 bis 3 phr und am meisten bevorzugt 0,7 bis 1,5 phr. Die Einheit phr steht für Gewichtsteile bezogen auf 100 Gewichtsteile an in der Kautschukmischung eingesetztem Kautschuk.

Für die Herstellung der erfindungsgemäßen Kautschukmischungen können Naturkautschuk und/oder Synthesekautschuke verwendet werden. Bevorzugte Synthesekautschuke sind beispielsweise
- BR: - Polybutadien
- ABR: - Butadien/Acrylsäure-C₁-C₄-alkylester-Copolymerisat
- CR: - Polychloropren
- IR: - Polyisopren
- SBR: - Styrol/Butadien-Copolymerisate mit Styrolgehalten von 1 - 60, vorzugsweise 20-50 Gew-%
- IIR: - Isobutylen/Isopren-Copolymerisate
- NBR: - Butadien/Acrylnitril-Copolymerisate mit Acrylnitrilgehalten von 5-60, vorzugsweise 10-50 Gew.-%
- HNBR: - teilhydrierter oder vollständig hydrierter NBR-Kautschuk
- EPDM: - Ethylen/Propylen/Dien-Copolymerisate
sowie Mischungen aus zwei oder mehr dieser Kautschuke.

Bevorzugt enthalten die erfindungsgemäßen Kautschukmischungen mindestens einen SBR-Kautschuk und mindestens einen BR-Kautschuk, besonders bevorzugt im Gewichtsverhältnis SBR:BR von 60:40 bis 90:10.

In einer vorteilhaften Ausführungsform enthalten die erfindungsgemäßen Kautschukmischungen zudem mindestens einen NR-Kautschuk. Besonders bevorzugt weisen sie wenigstens einen SBR-Kautschuk, wenigstens einen BR-Kautschuk und wenigstens einen NR-Kautschuk auf, wobei ganz besonders bevorzugt das Gewichtsverhältnis von SBR-Kautschuk zu BR-Kautschuk zu NR-Kautschuk 60 bis 85 : 10 bis 35 : 5 bis 20 beträgt.

Als schwefelhaltige Alkoxysilane für die erfindungsgemäßen Kautschukmischungen sind z.B. Bis-(triethoxysilylpropyl)tetrasulfan (z.B. Si 69 von Evonik) und Bis-(triethoxysilyl-propyl)disulfan (z.B. Si 75 von Evonik), 3-(Triethoxysilyl)-1-propanthiol, polyetherfunktionalisierte Mercaptosilane wie Si 363 von Evonik, thioesterfunktionalisierte Alkoxysilane wie NXT oder NXT Z von Momentive (früher GE) geeignet. Es können auch Mischungen der schwefelhaltigen Alkoxysilane eingesetzt werden. Flüssige schwefelhaltige Alkoxysilane können zur besseren Dosierbarkeit und/oder Dispergierbarkeit auf einem Träger aufgezogen sein (dry liquid). Der Wirkstoffgehalt liegt zwischen 30 und 70 Gew.-Teilen, bevorzugt 40 und 60 Gew.-Teilen je 100 Gew.-Teile dry liquid.

Der Anteil der schwefelhaltigen Alkoxysilane in den erfindungsgemäßen Kautschukmischungen beträgt vorzugsweise 2 bis 20 phr, besonders bevorzugt 3 bis 11 phr und ganz besonders bevorzugt 5 bis 8 phr, jeweils berechnet als 100%iger Wirkstoff. Vorzugsweise ist die Menge an schwefelhaltigem Alkoxysilan größer oder gleich der Menge an erfindungsgemäßen Polysulfiden der Formel (I). Besonders bevorzugt beträgt das Gewichtsverhältnis von schwefelhaltigem Alkoxysilan zu den erfindungsgemäßen Polysulfiden der Formel (I) 1,5:1 bis 20:1, ganz besonders bevorzugt 3:1 bis 15:1 und am meisten bevorzugt 5:1 bis 10:1.

Die erfindungsgemäß bevorzugte Kautschukmischung enthält zudem einen oder mehrere kieselsäurebasierende Füllstoffe. Vorzugsweise werden dafür folgende Stoffe verwendet:
- Kieselsäure, insbesondere Fällungskieselsäure oder pyrogene Kieselsäure, hergestellt z.B. durch Fällung von Lösungen von Silikaten oder Flammenhydrolyse von Siliziumhalogeniden mit spezifischen Oberflächen von 5 - 1000, vorzugsweise 20 - 400 m²/g (BET-Oberfläche) und mit Primärteilchengrößen von 10 - 400 nm. Die Kieselsäuren können gegebenenfalls auch als Mischoxide mit anderen Metalloxiden wie Al-, Mg-, Ca-, Ba-, Zn-, Zr-, Ti-Oxiden vorliegen.
- synthetische Silikate, wie Aluminiumsilikat, Erdalkalisilikate wie Magnesium- oder Calciumsilikat, mit BET-Oberflächen von 20 - 400 m²/g, vorzugsweise von 100 - 400 m²/g und Primärteilchengröße von 10 - 400 nm,
- natürliche Silikate, wie Kaolin und andere natürliche vorkommende Kieselsäuren,
- Glasfasern auch in Formen von Matten und Strängen,
- Mikroglaskugeln.

Es können selbstverständlich zusätzliche Füllstoffe verwendet werden. Insbesondere sind hierfür nach dem Flammruß, Furnace- oder Gasruß-Verfahren hergestellt Ruße geeignet, welche BET-Oberflächen von 20 - 200 m²/g besitzen, wie SAF-, ISAF-, IISAF-, HAF-, FEF- oder GPF-Ruße.

Der Gesamtgehalt an Füllstoffen beträgt bevorzugt 10 bis 200 phr, besonders bevorzugt 50 bis 160 phr und ganz besonders bevorzugt 60 bis 120 phr. Der Gewichtsanteil an kieselsäurebasierenden Füllstoffen beträgt mindest 10 %, vorzugsweise mindestens 20 %, besonders bevorzugt mindestens 50 % und am meisten bevorzugt mindestens 80 % des Gesamtfüllstoffgehalts.

Eine besonders bevorzugte Ausführungsform stellt die Kombination von Kieselsäure, Ruß und erfindungsgemäßen Polysulfiden der Formel (I) dar. Dabei kann das Verhältnis von Kieselsäure zu Ruß in beliebigen Grenzen variieren, wobei für die Anwendung in Reifen ein Kieselsäure : Ruß - Gewichtsverhältnis von 20 : 1 bis 1,5 : 1 bevorzugt ist.

In einer bevorzugten Ausführungsform enthalten die erfindungsgemäßen Kautschukmischungen auch einen oder mehrere Vernetzer. Hierfür sind insbesondere Schwefel-basierende oder peroxidische Vernetzer geeignet, wobei Schwefel-basierte Vernetzer besonders bevorzugt sind.

Als peroxidische Vernetzer werden vorzugsweise Bis(2,4-dichlorbenzyl)peroxid, Dibenzoylperoxid, Bis(4-chlorbenzoyl)peroxid, 1,1-Bis(t-butylperoxy)-3,3,5-trimethyl-cylohexan, tert-Butylperbenzoat, 2,2-Bis(t-butylperoxy)butan, 4,4-Di-tert-butylperoxynonylvalerat, Dicumylperoxid, 2,5-Dimethyl-2,5-di(tert-butylperoxy)hexan, tert-Butylcumylperoxid, 1,3-Bis(t-butylperoxyisopropyl)benzol, Di-tert-butylperoxid und 2,5-Dimethyl-2,5-di(tert-butylperoxy)-3-hexin eingesetzt.

Es kann vorteilhaft sein, neben diesen peroxidischen Vernetzern noch weitere Zusätze zu verwenden, mit deren Hilfe die Vernetzungsausbeute erhöht werden kann: Hierfür sind beispielsweise Triallylisocyanurat, Triallylcyanurat, Trimethylolpropantri(meth)acrylat, Triallyltrimellitat, Ethylenglycoldi(meth)acrylat, Butandioldi(meth)acrylat, Trimetylolpropan-tri(meth)acrylat, Zinkdiacrylat, Zinkdimethacrylat, 1,2-Polybutadien oder N,N'-m-Phenylen-dimaleinimid geeignet.

Als Vernetzer kann Schwefel in elementarer löslicher oder unlöslicher Form oder in Form von Schwefelspendern eingesetzt werden. Als Schwefelspender kommen beispielsweise Dimorpholyldisulfid (DTDM), 2-Morpholinodithiobenzothiazol (MBSS), Caprolactamdisulfid, Dipentamethylenthiuramtetrasulfid (DPTT) und Tetramethylthiuramdisulfid (TMTD) in Frage.

Grundsätzlich kann die Vernetzung der erfindungsgemäßen Kautschukmischungen mit Schwefel oder Schwefelspendern allein erfolgen, oder zusammen mit Vulkanisationsbeschleunigern, wofür z.B. Dithiocarbamate, Thiurame, Thiazole, Sulfenamide, Xanthogenate, bi- oder polycyclische Amine, Guanidinderivate, Dithiophosphate, Caprolactame und Thioharnstoffderivate geeignet sind. Weiterhin sind auch Zinkdiamindiisocyanat, Hexamethylentetramin, 1,3-Bis(citraconimidomethyl)benzol sowie zyklische Disulfane geeignet. Bevorzugt enthalten die erfindungsgemäßen Kautschukmischungen Schwefel-basierende Vernetzer und Vulkanisationsbeschleuniger.

Besonders bevorzugt werden als Vernetzungsmittel Schwefel, Magnesiumoxid und/oder Zinkoxid eingesetzt, denen die bekannten Vulkanisationsbeschleuniger, wie Merkaptobenzothiazole, Thiazolsulfenamide, Thiurame, Thiocarbamate, Guanidine, Xanthogenate und Thiophosphate zugesetzt werden.

Die Vernetzungsmittel und Vulkanisationsbeschleuniger werden bevorzugt in Mengen von 0,1 bis 10 phr, besonders bevorzugt von 0,1 bis 5 phr, eingesetzt.

Die erfindungsgemäßen Kautschukmischungen können weitere Kautschukhilfsmittel enthalten, wie Reaktionsbeschleuniger, Alterungsschutzmittel, Wärmestabilisatoren, Lichtschutzmittel, Antioxidantien, insbesondere Ozonschutzmittel, Flammschutzmittel, Verarbeitungshilfsmittel, Schlagzähfestigkeitsverbesserer, Weichmacher, Tackifier, Treibmittel, Farbstoffe, Pigmente, Wachse, Streckmittel, organische Säuren, Verzögerer, Metalloxide und Aktivatoren, insbesondere Triethanolamin, Polyethylenglykol, Hexantriol und Reversionsschutzmittel.

Diese Kautschukhilfsmittel werden in üblichen Mengen eingesetzt, die sich u. a. nach dem Verwendungszweck der Vulkanisate richten. Übliche Mengen sind 0,1 bis 30 phr.

Als Alterungsschutzmittel werden bevorzugt alkylierte Phenole, styrolisiertes Phenol, sterisch gehinderte Phenole wie 2,6-Di-tert.-butylphenol, 2,6-Di-tert-butyl-p-kresol (BHT), 2,6-Di-tert.-butyl-4-ethylphenol, estergruppenhaltige sterisch gehinderte Phenole, thioetherhaltige sterisch gehinderte Phenole, 2,2'-Methylen-bis-(4-methyl-6-tert-butylphenol) (BPH) sowie sterisch gehinderte Thiobisphenole verwendet.

Falls eine Verfärbung des Kautschuks ohne Bedeutung ist, können auch aminische Alterungsschutzmittel z. B. Mischungen aus Diaryl-p-phenylendiaminen (DTPD), octyliertes Diphenylamin (ODPA), Phenyl-α-naphthylamin (PAN), Phenyl-β-naphthylamin (PBN), vorzugsweise solche auf Phenylendiaminbasis, z. B. N-Isopropyl-N'-phenyl-p-phenylendiamin, N-1,3-Dimethylbutyl-N'-Phenyl-p-phenylendiamin (6PPD), N-1,4-Dimethylpentyl-N'-phenyl-p-phenylendiamin (7PPD), N,N'-bis-(1,4-Dimethylpentyl)-p-phenylendiamin (77PD) eingesetzt werden.

Weitere Alterungsschutzmittel sind Phosphite wie Tris-(nonylphenyl)phosphit, polymerisiertes 2,2,4-Trimethyl-1,2-dihydrochinolin (TMQ), 2-Mercaptobenzimidazol (MBI), Methyl-2-Mercaptobenzimidazol (MMBI), Zinkmethylmercaptobenzimidazol (ZMMBI), welche meist in Kombination mit obigen phenolischen Alterungsschutzmitteln eingesetzt werden. TMQ, MBI und MMBI werden vor allem für NBR-Kautschuke verwendet, welche peroxidisch vulkanisiert werden.

Die Ozonbeständigkeit kann durch Antioxidantien wie beispielsweise N-1,3-Dimethylbutyl-N'-phenyl-p-phenylendiamin (6PPD), N-1,4-Dimethylpentyl-N'-phenyl-p-phenylendiamin (7PPD), N,N'-Bis- (1,4-dimethylpentyl)-p-phenylendiamin (77PD), Enolether oder cyclische Acetale. verbessert werden.

Verarbeitungshilfsmittel sollen zwischen den Kautschuk-Partikeln wirksam werden und Reibungskräften beim Mischen, Plastifizieren und Verformen entgegenwirken. Als Verarbeitungshilfsmittel können die erfindungsgemäßen Kautschukmischungen alle für die Verarbeitung von Kunststoffen üblichen Gleitmittel enthalten, wie beispielsweise Kohlenwasserstoffe, wie Öle, Paraffine und PE-Wachse, Fettalkohole mit 6 bis 20 C-Atomen, Ketone, Carbonsäuren, wie Fettsäuren und Montansäuren, oxidiertes PE-Wachs, Metallsalze von Carbonsäuren, Carbonsäureamide sowie Carbonsäureester, beispielsweise mit den Alkoholen Ethanol, Fettalkoholen, Glycerin, Ethandiol, Pentaerythrit und langkettigen Carbonsäuren als Säurekomponente.

Um die Entflammbarkeit zu vermindern und die Rauchentwicklung beim Verbrennen zu verringern, kann die erfindungsgemäße Kautschukmischungszusammensetzung auch Flammschutzmittel enthalten. Hierfür werden beispielsweise Antimontrioxid, Phosphorsäureester, Chlorparaffin, Aluminiumhydroxid, Borverbindungen, Zinkverbindungen, Molybdäntrioxid, Ferrocen, Calciumcarbonat oder Magnesiumcarbonat verwendet.

Vor der Vernetzung können dem Kautschukvulkanisat auch weitere Kunststoffe beigefügt werden, welche beispielsweise als polymere Verarbeitungshilfsmittel oder Schlagzähigkeitsverbesserer wirken. Diese Kunststoffe werden bevorzugt gewählt aus der Gruppe bestehend aus den Homo- und Copolymeren auf Basis von Ethylen, Propylen, Butadien, Styrol, Vinylacetat, Vinylchlorid, Glycidylacrylat, Glycidylmethacrylat, Acrylaten und Methacrylaten mit Alkoholkomponenten von verzweigten oder unverzweigten C₁- bis C₁₀-Alkoholen, wobei Polyacrylate mit gleichen oder verschiedenen Alkoholresten aus der Gruppe der C₄- bis C₈-Alkohole, insbesondere des Butanols, Hexanols, Octanols und 2-Ethylhexanols, Polymethylmethacrylat, Methylmethacrylat-Butylacrylat-Copolymere, Methylmethacrylat-Butylmethacrylat-Copolymere, Ethylen-Vinylacetat-Copolymere, chloriertes Polyethylen, Ethylen-Propylen-Copolymere, Ethylen-Propylen-Dien-Copolymere besonders bevorzugt sind.

In einer bevorzugten Ausführungsform enthält die erfindungsgemäße Kautschukmischung 0,1 bis 15 phr des Reversionsschutzmittels 1,6-Bis(N,N-dibenzylthiocarbamoyl-dithio)hexan (CAS-Nr.: 151900-44-6), wodurch eine Verringerung von tan δ (60 °C), d.h. des Rollwiderstands ermöglicht wird, die Abriebwerte verbessert und Anvulkanisationszeit und Ausvulkanisationszeit verkürzt werden.

Bevorzugt zeichnen sich die erfindungsgemäßen Kautschukmischungen dadurch aus, dass ein daraus bei 170°C/t95 geheiztes Vulkanisat einen Verlustfaktor tan δ bei 60°C von < 0,16, besonders bevorzugt < 0,12, insbesondere < 0,10 und gleichzeitig eine Härte Shore A bei 23°C von > 66 aufweist. In Kombination damit können durch die erfindungsgemäßen Kautschukmischungen auch eine Ausvulkanisationszeit von kleiner 2000 Sekunden und ein Modul 300-Wert von > 15 MPa erreicht werden.

Ein weiterer Gegenstand der vorliegenden Erfindung ist ein Verfahren zur Herstellung von Kautschukmischungen, durch Mischen von mindestens einem Kautschuk mit mindestens einem kieselsäurebasierenden Füllstoff, einem schwefelhaltigen Alkoxysilan und mindestens einer erfindungsgemäßen Polysulfid-Mischung. Vorzugsweise werden dabei 10 bis 150 phr, besonders bevorzugt 30 bis 120 phr und ganz besonders bevorzugt 50 bis 100 phr Füllstoff, 0,1 bis 15 phr, besonders bevorzugt 0,3 bis 7 phr, ganz besonders bevorzugt 0,5 bis 3 phr und am meisten bevorzugt 0,7 bis 1,5 phr erfindungsgemäßer Polysulfide der Formel (I) sowie 2 bis 20 phr, besonders bevorzugt 3-11 phr und ganz besonders bevorzugt 5 bis 8 phr des schwefelhaltigen Alkoxysilans eingesetzt. Weiterhin können im Mischverfahren die oben genannten zusätzlichen Füllstoffe, Vernetzer, Vulkanisationsbeschleuniger und Kautschukhilfsmittel, vorzugsweise in den oben angegebenen Mengen zugesetzt werden.

Bei dem mehrstufigen Mischverfahren erfolgt die Zugabe der erfindungsgemäßen Polysulfide der Formel (I) bevorzugt im ersten Teil des Mischprozesses und die Zugabe von einem oder mehreren Vernetzern, insbesondere Schwefel, und ggf. Vulkanisationsbeschleuniger in einer späteren Mischstufe. Die Temperatur der Kautschukmasse beträgt dabei vorzugsweise 100 bis 200 °C, besonders bevorzugt 120°C bis 170 °C. Die Scherraten bei der Mischung betragen 1 bis 1000 sec⁻¹, bevorzugt 1 bis 100 sec⁻¹. In einer bevorzugten Ausführungsform wird die Kautschukmischung nach der ersten Mischstufe abgekühlt und der Vernetzer und ggf. Vernetzungsbeschleuniger und/oder Zusätze mit deren Hilfe die Vernetzungsausbeute erhöht wird, in einer späteren Mischstufe bei < 140°C, vorzugsweise < 100°C zugegeben. Die Zugabe der erfindungsgemäßen Polysulfide der Formel (I) in einer späteren Mischstufe und bei tieferen Temperaturen wie 40 bis 100°C ist ebenfalls möglich z.B. zusammen mit Schwefel und Vernetzungsbeschleuniger.

Die Abmischungen des Kautschuks mit dem Füllstoff und den erfindungsgemäßen Polysulfiden der Formel (I) kann in üblichen Mischaggregaten, wie Walzen, Innenmischer und Mischextruder, durchgeführt werden.

Die optionale Zugabe von 1,6-Bis(N,N-dibenzylthiocarbamoyldithio)hexan findet vorzugsweise in der ersten Stufe des mehrstufigen Mischverfahrens statt.

Ein weiterer Gegenstand der vorliegenden Erfindung ist ein Verfahren zur Vulkanisation der erfindungsgemäßen Kautschukmischungen, welche bevorzugt bei Massetemperaturen von 100 bis 200 °C, besonders bevorzugt bei 130 bis 180 °C durchgeführt wird. In einer bevorzugten Ausführungsform geschieht die Vulkanisation bei einem Druck von 10 bis 200 bar.

Die vorliegende Erfindung umfasst auch Kautschukvulkanisate erhältlich durch Vulkanisation der erfindungsgemäßen Kautschukmischungen. Diese Vulkanisate weisen insbesondere bei Anwendung in Reifen die Vorteile ein hervorragendes Eigenschaftsprofil und einen unerwartet niedrigen Rollwiderstand auf.

Die erfindungsgemäßen Kautschukvulkanisate eignen sich zur Herstellung von Formkörpern mit verbesserten Eigenschaften, z.B. für die Herstellung von Kabelmäntel, Schläuchen, Treibriemen, Förderbändern, Walzenbelägen, Reifen, Schuhsohlen, Dichtungsringen und Dämpfungselementen.

Das erfindungsgemäße Kautschukvulkanisat kann zudem zur Herstellung von Schaumstoffen verwendet werden. Dazu werden ihr chemisch oder physikalisch wirkende Treibmittel zugefügt. Als chemisch wirkende Treibmittel kommen alle für diesen Zweck bekannten Substanzen in Betracht, wie beispielsweise Azodicarbonamid, p-Toluolsulfonylhydrazid, 4,4'-Oxybis(benzolsulfohydrazid), p-Toluolsulfonylsemicarbazid, 5-Phenyltetrazol, N,N'-Dinitroso-pentamethylentetramin, Zinkcarbonat oder Natriumhydrogencarbonat sowie diese Substanzen enthaltende Mischungen. Als physikalisch wirkende Treibmittel sind beispielsweise Kohlendioxid oder Halogenkohlenwasserstoffe geeignet.

Ein weiterer Gegenstand der vorliegenden Erfindung ist die Verwendung der erfindungsgemäßen Polysulfide der Formel (I) zur Herstellung von Kautschukmischungen, und deren Vulkanisaten. Bevorzugt enthalten die Kautschukmischungen zumindest jeweils einen Kautschuk, einen Füllstoff und die erfindungsgemäßen Polysulfide der Formel (I), besonders bevorzugt zumindest jeweils einen Kautschuk, ein schwefelhaltiges Alkoxysilan, einen kieselsäurebasierenden Füllstoff und die erfindungsgemäßen Polysulfide der Formel (I).

Überraschenderweise wurde gefunden, dass Mischungen enthaltend zumindest ein schwefelhaltiges Alkoxysilan, insbesondere Bis-(triethoxysilylpropyl)tetrasulfan, Bis-(triethoxysilylpropyl)disulfan, 3-(Triethoxysilyl)-1-propanthiol, polyetherfunktionalisiertes Mercaptosilan oder thioesterfunktionalisiertes Alkoxysilan und erfindungsgemäße Polysulfide der Formel (I) trotz der reaktiven Gruppen eine ausreichende Verträglichkeit der Komponenten aufweisen, wodurch eine homogene Einbringung in Kautschukmischungen und eine exakte Dosierung im gewünschten Verhältnis ermöglicht wird.

Daher umfasst die vorliegende Erfindung auch solche, als Additiv verwendbare Mischungen, sowie die Verwendung von schwelfelhaltigen Alkoxysilanen, insbesondere Bis-(triethoxysilylpropyl)tetrasulfan, Bis-(triethoxysilylpropyl)disulfan, 3-(Triethoxysilyl)-1-propanthiol, polyetherfunktionalisierten Mercaptosilanen oder thioesterfunktionalisierten Alkoxysilanen, und erfindungsgemäßen Polysulfiden der Formel (I) zur Herstellung dieser Mischungen. Vorzugsweise ist das Gewichtsverhältnis von Alkoxysilan, insbesondere von Bis-(triethoxysilylpropyl)tetrasulfan und/oder von Bis-(triethoxysilylpropyl)disulfan zu erfindungsgemäßen Polysulfiden der Formel (I) 1,5:1 bis 20:1, besonders bevorzugt 3:1 bis 15:1 und am meisten bevorzugt 5:1 bis 10:1.

### Bestimmung der Eigenschaften von Kautschukmischung bzw. Vulkanisaten:

### Mooney-Viskositätsmessung:

Die Viskosität lässt sich aus der Kraft, die Kautschuke (und Kautschukmischungen) ihrer Verarbeitung entgegensetzen, direkt bestimmen. Beim Scherscheibenviskosimeter nach Mooney wird eine geriffelte Scheibe oben und unten mit Probensubstanz umschlossen und in einer beheizbaren Kammer mit etwa zwei Umdrehungen in der Minute bewegt. Die hierzu erforderliche Kraft wird als Drehmoment gemessen und entspricht der jeweiligen Viskosität. Die Probe wird in der Regel eine Minute lang auf 100°C vorgewärmt; die Messung dauert weitere 4 Minuten, wobei die Temperatur konstant gehalten wird. Die Viskosität wird zusammen mit den jeweiligen Prüfbedingungen angegeben, beispielsweise ML (1+4) 100°C (Mooney viscosity, large rotor, Vorwärmzeit und Prüfzeit in Minuten, Prüftemperatur).

### Scorch-Verhalten (Anvulkanisationszeit t 5):

Des Weiteren kann mit der gleichen Prüfung wie oben beschrieben auch das Scorch-Verhalten einer Mischung gemessen werden. Die gewählte Temperatur betrug 130°C. Der Rotor läuft solang, bis der Drehmomentswert nach Durchlaufen eines Minimums auf 5 Mooney-Einheiten relativ zum Minimumswert angestiegen ist (t5). Je größer der Wert ist (Einheit Sekunden), umso langsamer findet die Anvulkanisation statt. In der Praxis ist meist eine Anvulkanisationszeit von mehr als 300 Sekunden vorteilhaft.

### Rheometer (Vulkameter) Ausvulkanisationszeit 170°C/t95:

Der Vulkanisationsverlauf am MDR (moving die rheometer) und dessen analytischen Daten werden an einem Monsanto-Rheometer MDR 2000 nach ASTM D5289-95 gemessen. Als Ausvulkanisationszeit, wird die Zeit bestimmt, bei der 95% des Kautschuks vernetzt ist. Die gewählte Temperatur betrug 170°C.

### Bestimmung der Härte:

Zur Bestimmung der Härte der erfindungsgemäßen Kautschukmischung wurden 6 mm starke Walzfelle aus der Kautschukmischung gemäß Rezepturen der Tabelle 1 hergestellt. Aus den Walzfellen wurden Prüfkörper mit 35 mm Durchmesser geschnitten, deren Shore-Härte A mittels eines digitalen Shore-Härte-Testers (Zwick GmbH & Co. KG, Ulm) bestimmt wurden. Die Härte eines Kautschukvulkanisates gibt einen ersten Hinweis über dessen Steifigkeit wieder.

### Zugversuch:

Der Zugversuch dient direkt zur Ermittlung der Belastungsgrenzen eines Elastomers und erfolgt nach DIN 53504. Die Längenausdehnung beim Bruch wird auf die Ausgangslänge bezogen und entspricht der Bruchdehnung. Weiterhin wird auch die Kraft beim Erreichen bestimmter Dehnungsstufen, meist 50, 100, 200 und 300% bestimmt und als Spannungswert ausgedrückt (Zugfestigkeit bei der angegebenen Dehnung von 300% oder Modul 300).

### Dyn. Dämpfung:

Dynamische Prüfverfahren werden zur Charakterisierung des Verformungsverhaltens von Elastomeren unter periodisch veränderten Belastungen verwendet. Eine von außen angebrachte Spannung verändert die Konformation der Polymerkette. Der Verlustfaktor tan δ wird dabei indirekt über das Verhältnis zwischen Verlustmodul G" und Speichermodul G' bestimmt. Der Verlustfaktor tan δ bei 60 °C geht mit dem Rollwiderstand einher und sollte möglichst niedrig sein.

### Abrieb:

Der Abrieb gibt einen Hinweis auf die Abnutzung und damit auf die Lebensdauer eines Produktes. Der Abrieb wurde nach DIN 53516 bestimmt. Aus ökonomischen und ökologischen Gründen ist ein niedriger Wert anzustreben.

### Beispiel 1

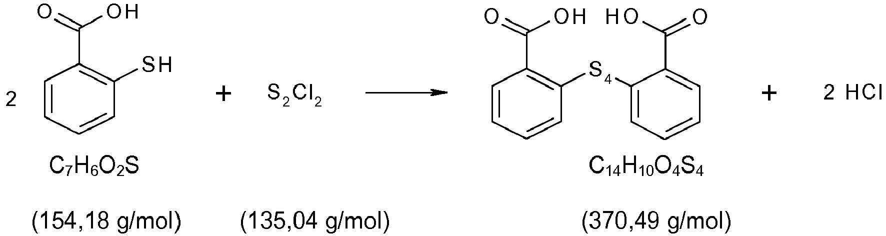

Apparatur: 2000ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr
Vorlage: 118,0 g = 0,75 mol Mercaptobenzoesäure (Merck, ≥ 98%), 900 ml Toluol (p.A., Fa. Merck über Molekularsieb getrocknet)
- Zulauf:51,15 g = 0,375: mol Dischwefeldichlorid (≥ 99%, Fa. Merck)

In der stickstoffgespülten Apparatur werden getrocknetes Toluol und Mercaptobenzoesäure vorgelegt. In die vorliegende Suspension wird nun das Dischwefeldichlorid unter Stickstoffdurchleitung bei einer Temperatur von 0-5°C innerhalb von ca. einer 1h zugetropft. Die Dosiergeschwindigkeit ist so einzustellen, dass eine Temperatur von 5°C nicht überschritten wird. Nach beendeter Reaktion wird unter Stickstoffdurchleitung bei Raumtemperatur über Nacht nachgerührt. Anschließend wird die Reaktionslösung über eine D4-Fritte abgesaugt und 2x mit ca. 200ml getrocknetem Toluol nachgewaschen. Das Produkt wird bei Raumtemperatur (ca. 25°C) im Vakuumtrockenschrank getrocknet.
Ausbeute: 144,6 g (104,1%) einer Polysulfidmischung der idealisierten Formel

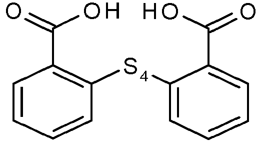

| Elementaranalyse: | | | | |
|---|---|---|---|---|
| C: 45,0% | H: 3,0% | O: 16,7% | S: 33,6% | Cl: 1,4% |

Das Produkt riecht sehr intensiv.

Die Mischung von 10g Polysulfidmischung mit 100ml Wasser zeigte nach 30min Rückfluss und Abkühlen auf 25°C einen pH von 1,2 und eine Leitfähigkeit von 14mS/cm.

### Beispiel 2

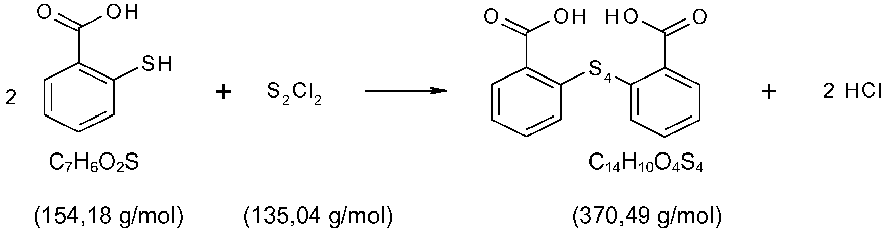

Apparatur: 2000ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr
Vorlage: 118,0 g = 0,75 mol Mercaptobenzoesäure (Merck, ≥ 98%), 900 ml Toluol (p.A., Fa. Merck über Molekularsieb getrocknet)
Zulauf:51,15 g = 0,375 mol Dischwefeldichlorid (≥ 99%, Fa. Merck)

In der stickstoffgespülten Apparatur werden getrocknetes Toluol und Mercaptobenzoesäure vorgelegt. In die vorliegende Suspension wird nun das Dischwefeldichlorid unter Stickstoffdurchleitung bei einer Temperatur von 20-25°C innerhalb von ca. 30min zugetropft. Die Dosiergeschwindigkeit ist so einzustellen, dass eine Temperatur von 25°C nicht überschritten wird. Nach beendeter Reaktion wird unter Stickstoffdurchleitung bei 20-25°C 1h nachgerührt. Dann werden 20ml VE-Wasser und 180ml Toluol zugegeben und unter Stickstoffdurchleitung für 2h zum Rückfluss erhitzt. Anschließend werden mit Stickstoffdurchleitung ca. 200ml unter Normaldruck abdestilliert.

Der Ansatz wird auf Raumtemperatur abgekühlt. Anschließend wird die Reaktionslösung über eine D4-Fritte abgesaugt und 2x mit ca. 200ml getrocknetem Toluol nachgewaschen. Das Produkt wird bei Raumtemperatur (ca. 25°C) im Vakuumtrockenschrank getrocknet.

Ausbeute: 139,5 g (100,4%) einer Polysulfidmischung der idealisierten Formel

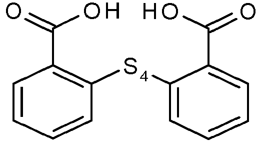

| Elementaranalyse: | | | | |
|---|---|---|---|---|
| C:45,2% | H: 2,7% | O: 18,1% | S: 33,6% | Cl: 0,05% |

Das Produkt wurde durch RP-HPLC und Time-of-Flight Mass Spectrometry (TOF MS) analysiert.

Die Prozentangaben zu den Verbindungen der Formel (I) ergeben sich direkt aus den Flächenprozentangaben der HPLC-Messung mit UV-Detektor.
< 1% Verbindung (II), 1% Verbindung (III), 97% Verbindung (IV), 1% Verbindung (V), < 1 % Verbindung (VI).

HPLC-Gerät: Agilent 1100 Series mit Degaser, binärer Pumpe, Säulenofen, variablem Wellenlängendetektor und Autosampler
- Stationäre Phase:: Inertsil ODS-3, Teilchendurchmesser 3 µm
- Säulenlänge:: 150 mm
- Säuleninnendurchmesser:: 2,1 mm
- Mobile Phase: A: 100% Wasser + 25 mmol Ammoniumacetat
B: 95% Methanol : 5% Wasser + 25 mmol Ammoniumacetat

### Elutionsprogram:

| Zeit (min) | Eluent A (Vol%) | Eluent B (Vol%) |
|---|---|---|
| 0 | 80 | 20 |
| 5 | 80 | 20 |
| 30 | 1 | 99 |
| 60 | 1 | 99 |
| 62 | 80 | 20 |

| | | |
|---|---|---|
| Säulentemperatur: | 40°C | |
| Fließgeschwindigkeit: | 0,3 ml/min | |
| Laufzeit: | 72 min | |
| Injektionsvolumen: | 5 µl | |
| Wellenlänge des UV-Detektors: | 225 nm | |

Eine 50 mg Probe zu analysierendes Produkt wurden in einem 50 ml Messkolben eingewogen, durch Zugabe von ca. 10ml Tetrahydrofuran gelöst und mit Tetrahydrofuran bis zur Eichmarke aufgefüllt.

Das Produkt hat einen schwachen Eigengeruch.

Die Mischung von 10g Polysulfidmischung mit 100ml Wasser zeigte nach 30min Rückfluss und Abkühlen auf 25°C einen pH von 3,4 und eine Leitfähigkeit von 0,2 mS/cm.

### Beispiel 3

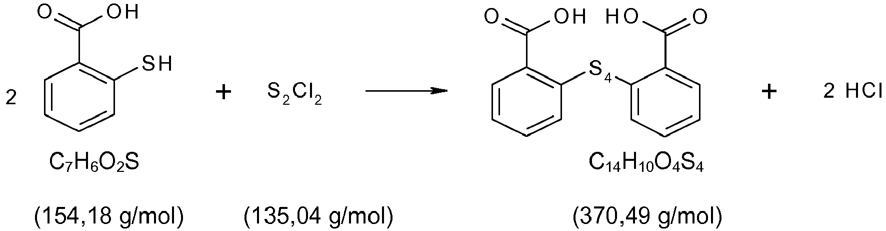

Apparatur: 2000ml Vierhalskolben mit Thermometer, Tropftrichter mit Druckausgleich, Rückflusskühler mit Gasableitungsansatz (Blasenzähler) und Schlauch, Rührwerk, Gaseinleitungsrohr
Vorlage: 236,0 g = 1,5 mol Mercaptobenzoesäure (Merck, ≥ 98%), 1000 ml Toluol (p.A., Fa. Merck über Molekularsieb getrocknet)
Zulauf:102,3 g = 0,75mol Dischwefeldichlorid (≥ 99%, Fa. Merck)

In der stickstoffgespülten Apparatur werden getrocknetes Toluol und Mercaptobenzoesäure vorgelegt. In die vorliegende Suspension wird nun das Dischwefeldichlorid unter Stickstoffdurchleitung bei einer Temperatur von 20-25°C innerhalb von ca. 30min zugetropft. Die Dosiergeschwindigkeit ist so einzustellen, dass eine Temperatur von 25°C nicht überschritten wird. Nach beendeter Reaktion wird unter Stickstoffdurchleitung bei 20-25°C 1h nachgerührt. Dann werden 100ml VE-Wasser zugegeben und unter Stickstoffdurchleitung für 4h zum Rückfluss erhitzt.

Der Ansatz wird auf Raumtemperatur abgekühlt. Anschließend wird die Reaktionslösung über eine D4-Fritte abgesaugt und 2x mit ca. 300ml Toluol nachgewaschen. Das Produkt wird bei ca. 50°C im Vakuumtrockenschrank getrocknet.
Ausbeute: 279g (100,4%) einer Polysulfidmischung der idealisierten Formel

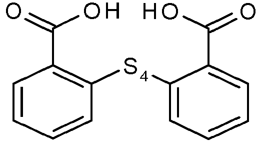

| Elementaranalyse: | | | | |
|---|---|---|---|---|
| C:45,3% | H: 2,9% | O: 17,4% | S: 34,5% | Cl: 0,02% |

Das Produkt wurde durch RP-HPLC und Time-of-Flight Mass Spectrometry (TOF MS) analysiert.

Die Prozentangaben zu den Verbindungen der Formel (I) ergeben sich direkt aus den Flächenprozentangaben der HPLC-Messung mit UV-Detektor.
< 1 % Verbindung (II), < 1% Verbindung (III), 97% Verbindung (IV), 1% Verbindung (V), < 1% Verbindung (VI).

Das Produkt hat einen schwachen Eigengeruch.

Die Mischung von 10g Polysulfidmischung mit 100ml Wasser zeigte nach 30min Rückfluss und Abkühlen auf 25°C einen pH von 3,5 und eine Leitfähigkeit von 0,1 mS/cm.

### Beispiel 4

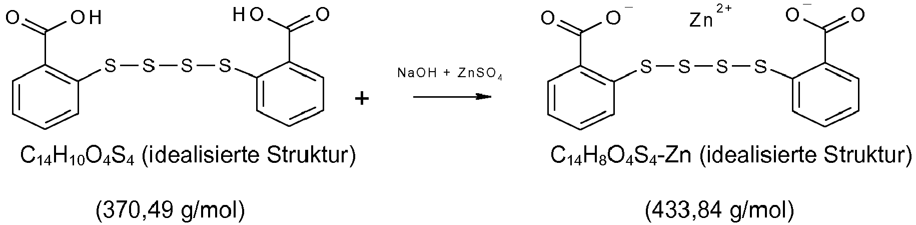

Apparatur: 2000ml Vierhalskolben mit Thermometer, Tropftrichter mit Druckausgleich, Rückflusskühler mit Gasableitungsansatz (Blasenzähler) und Schlauch, Rührwerk, pH-Elektrode

| | |
|---|---|
| Vorlage: 74,1 g | Polysulfidmischung Beispiel 1, 700 ml Wasser |
| Zulauf: 35,9 g | = 0,2 mol ZnSO₄ x H₂O (Fa. Aldrich 100%) gelöst in 300 ml Wasser |
| Zulauf:160,0 g | = 0,4 mol NaOH-Lösung (10%ig) |

In der stickstoffgespülten Apparatur werden das Wasser und die Polysulfidmischung aus Beispiel 1 vorgelegt und auf 95 - 100°C erwärmt. In den vorliegenden Ansatz wird nun die ZnSO₄-Lösung unter Stickstoffüberleitung bei einer Temperatur von 95 - 100°C innerhalb von ca. 1h zugetropft. Der Ansatz wird 1h nachgerührt. Anschließend wird bei 95 - 100°C die NaOH-Lösung in ca. 1h zudosiert. 1h wird bei ca. 100°C nachgerührt. Das Produkt wird nach dem Abkühlen über eine D4-Fritte abgesaugt und mit 500ml Wasserportionen so lange gewaschen, bis die Leitfähigkeit des Waschwassers < 0,3 mS/cm ist. Das Produkt wird bei 50°C im Vakuumtrockenschrank getrocknet.
Ausbeute: 79,3 g (91,4%) einer Polysulfidmischung der idealisierten Formel

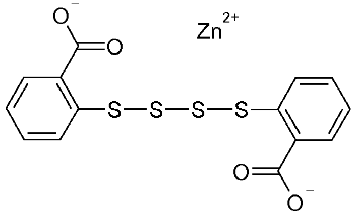

| Elementaranalyse: | | | | | |
|---|---|---|---|---|---|
| C: 37,8% | H: 2,1% | O: 16,0% | S: 29,7% | Zn: 16% | Cl: 110ppm |

Das Produkt hat einen schwachen Eigengeruch.

Die Mischung von 10g Polysulfidmischung mit 100ml Wasser zeigte nach 30min Rückfluss und Abkühlen auf 25°C einen pH von 5,9 und eine Leitfähigkeit von 0,6 mS/cm.

### Beispiel 5

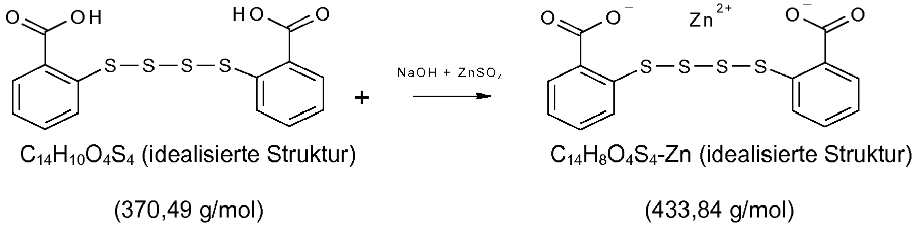

Apparatur: 2000ml Vierhalskolben mit Thermometer, Tropftrichter mit Druckausgleich, Rückflusskühler mit Gasableitungsansatz (Blasenzähler) und Schlauch, Rührwerk, pH-Elektrode
- Vorlage: 74,1 g: Polysulfidmischung Beispiel 1, 700 ml Wasser
- Zulauf: 35,9 g: = 0,2 mol ZnSO₄ x H₂O (Fa. Aldrich 100%) gelöst in 300 ml Wasser
- Zulauf:160,0 g: = 0,4 mol NaOH-Lösung (10%ig)

In der stickstoffgespülten Apparatur werden das Wasser und die Polysulfidmischung aus Beispiel 1 vorgelegt bei 20 - 25°C. In den vorliegenden Ansatz wird nun die ZnSO₄-Lösung unter Stickstoffüberleitung bei einer Temperatur von 20 - 25°C innerhalb von ca. 1h zugetropft. Der Ansatz wird 1h nachgerührt. Anschließend wird bei 20 - 25°C die NaOH-Lösung in ca. 1h zudosiert. Dann wird zum schwachen Rückfluss erwärmt und 1h bei ca. 100°C nachgerührt. Das Produkt wird nach dem Abkühlen über eine D4-Fritte abgesaugt und mit 500ml Wasserportionen so lange gewaschen, bis die Leitfähigkeit des Waschwassers < 0,3 mS/cm ist. Das Produkt wird bei 50°C im Vakuumtrockenschrank getrocknet.
- Ausbeute: 79,0 g: (91,0%) einer Polysulfidmischung der idealisierten Formel

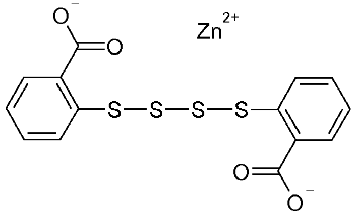

| Elementaranalyse: | | | | | |
|---|---|---|---|---|---|
| C: 38,5 % | H: 2,2% | O: 16,0% | S: 29,7% | Zn: 14% | Cl: 80ppm |

Das Produkt hat einen schwachen Eigengeruch.

Die Mischung von 10g Polysulfidmischung mit 100ml Wasser zeigte nach 30min Rückfluss und Abkühlen auf 25°C einen pH von 6,0 und eine Leitfähigkeit von 0,4 mS/cm.

### Herstellung von Kautschukmischungen und Kautschukvulkanisaten

Die in Tabelle 1 aufgeführten Kautschuk-Rezepturen wurden jeweils gemäß nachfolgend beschriebenen, mehrstufigen Verfahren gemischt.

### 1. Mischstufe:

■ BUNA^{®} CB 24 und BUNA^{®} VSL 5025-2 wurde in einem Innenmischer vorgelegt und ca. 30 Sekunden gemischt
■ Zugabe zwei Drittel VULKASIL^{®} S, zwei Drittel SI^{®} 69, zwei Drittel der Gesamtmenge an erfindungsgemäßen Polysulfide der Formel (I), mischen für ca. 60 Sekunden
■ Zugabe ein Drittel VULKASIL^{®} S, ein Drittel SI^{®} 69, ein Drittel der Gesamtmenge an erfindungsgemäßer Polysulfide der Formel (I) sowie TUDALEN 1849-1, mischen ca. 60 Sekunden
Zugabe von CORAX^{®} N 339, EDENOR^{®} C 18 98-100, VULKANOX^{®} 4020/LG, VULKANOX^{®} HS/LG, ZINKWEISS ROTSIEGEL sowie ANTILUX^{®} 654, mischen für ca. 60 Sekunden. Die Mischung erfolgte bei einer Temperatur von 150°C.

### 2. Mischstufe:

Nach Abschluss der ersten Mischstufe wurde das Mischstück von einem nachgeschalteten Walzwerk aufgenommen und zu einer Platte ausgeformt und für 24 Stunden bei Raumtemperatur gelagert. Die Verarbeitungstemperaturen lagen hierbei unter 60°C.

### 3. Mischstufe:

In der dritten Mischstufe erfolgte ein Nachzwicken bei 150°C in einem Kneter.

### 4. Mischstufe:

Zugabe der Zusatzstoffe MAHLSCHWEFEL 90/95 CHANCEL, VULKACIT^{®} CZ/C, VULKACIT^{®} D/C auf einer Walze bei Temperaturen unter 80°C.

Die Kautschukmischungen wurden anschließend bei 170°C ausvulkanisiert. Die Eigenschaften der hergestellten Kautschukzubereitungen und deren Vulkanisate sind in Tabelle 2 angegeben.

**Tabelle 1: Kautschuk-Rezeptur**

| | Referenz | Kautschuk-rezeptur 1 | Kautschuk-rezeptur 2 | Kautschuk-rezeptur 3 | Kautschuk-rezeptur 4 | Kautschuk-rezeptur 5 |
|---|---|---|---|---|---|---|
| BUNA CB 24 | 30 | 30 | 30 | 30 | 30 | 30 |
| BUNA VSL 5025-2 | 96 | 96 | 96 | 96 | 96 | 96 |
| CORAX N 339 | 6,4 | 6,4 | 6,4 | 6,4 | 6,4 | 6,4 |
| VULKASIL S | 80 | 80 | 80 | 80 | 80 | 80 |
| TUDALEN 1849-1 | 8 | 8 | 8 | 8 | 8 | 8 |
| EDENOR C 18 98-100 | 1 | 1 | 1 | 1 | 1 | 1 |
| VULKANOX 4020/LG | 1 | 1 | 1 | 1 | 1 | 1 |
| VULKANOX HS/LG | 1 | 1 | 1 | 1 | 1 | 1 |
| ZINKWEISS ROTSIEGEL | 2,5 | 2,5 | 2,5 | 2,5 | 2,5 | 2,5 |
| ANTILUX 654 | 1,5 | 1,5 | 1,5 | 1,5 | 1,5 | 1,5 |
| SI 69 | 6,4 | 6,4 | 6,4 | 6,4 | 6,4 | 6,4 |
| VULKACIT D/C | 2 | 2 | 2 | 2 | 2 | 2 |
| VULKACIT CZ/C | 1,5 | 1,5 | 1,5 | 1,5 | 1,5 | 1,5 |
| MAHLSCHWEFEL 90/95 CHANCEL | 1,5 | 1,5 | 1,5 | 1,5 | 1,5 | 1,5 |
| Verbindung Beispiel 1 | | 1 | | | | |
| Verbindung Beispiel 2 | | | 1 | | | |
| Verbindung Beispiel 3 | | | | 1 | | |
| Verbindung Beispiel 4 | | | | | 1 | |
| Verbindung Beispiel 5 | | | | | | 1 |

Mengenangaben in phr (Gewichtsteile pro 100 Teile Kautschuk)

| **Handelsname** | **Erläuterung** | **Hersteller/Vertrieb** |
|---|---|---|
| BUNA CB 24 | BR | Lanxess Deutschland GmbH |
| BUNA VSL 5025-2 | SBR | Lanxess Deutschland GmbH |
| CORAX N 339 | Ruß | Degussa-Evonik GmbH |
| VULKASIL S | Kieselsäure | Lanxess Deutschland GmbH |
| TUDALEN 1849-1 | Mineralöl | Hansen&Rosenthal KG |
| EDENOR C 18 98-100 | Stearinsäure | Cognis Deutschland GmbH |
| VULKANOX 4020/LG | N-1,3-Dimethylbutyl-N-phenyl-p-phenylendiamin | Lanxess Deutschland GmbH |
| VULKANOX HS/LG | 2,2,4-Trimethyl-1,2-dihydrochinolin polymerisiert | Lanxess Deutschland GmbH |
| ZINKWEISS ROTSIEGEL | Zinkoxid | Grillo Zinkoxid GmbH |
| ANTILUX 654 | Lichtschutzwachs | RheinChemie Rheinau GmbH |
| SI 69 | Bis(triethoxysilylpropyl)tetrasulfid | Evonik Industries |
| VULKACIT D/C | 1,3-Diphenylguanidin | Lanxess Deutschland GmbH |
| VULKACIT CZ/C | N-cyclohexyl-2-benzothiazolsulfenamid | Lanxess Deutschland GmbH |
| MAHLSCHWEFEL 90/95 CHANCEL | Schwefel | Solvay Deutschland GmbH |

**Tabelle 2: Zusammenstellung der Ergebnisse**

| **Parameter** | **Einheit** | **DIN** | **Referenz** | **Kautschukrezeptur 1** | **Kautschukrezeptur 2** |
|---|---|---|---|---|---|
| Mooney Viskosität (ML 1+4) | [ME] | 53523 | 95 | 92 | 84 |
| Mooney Anvulkanisation bei 130°C (t5) | sec | ASTM D 5289-95 | 1253 | 1228 | 1020 |
| Ausvulkanisation bei 170°C / t95 | sec | 53529 | 1417 | 1315 | 1475 |
| Shore A Härte bei 23°C | [Shore A] | 53505 | 66 | 73 | 67 |
| Modul 300 | MPa | 53504 | 15 | 18 | 16 |
| Bruchdehnung | % | 53504 | 349 | 308 | 361 |
| Zugfestigkeit | MPa | 53504 | 19 | 18 | 20 |
| Abrieb | mm³ | 53516 | 74 | 93 | 83 |
| Rollwiderstand (tan δ (60 °C)) | - | | 0,133 | 0,154 | 0,093 |

Die getesteten Vulkanisate zeigen im Vergleich zur Referenz erhöhte Härtewerte und Modul 300 Werte. Kautschukrezeptur 2 zeigt exzellenten Rollwiderstand, verbesserte Bruchdehnung und Zugfestigkeit und verbesserte Mooney-Viskosität.

## Patentansprüche

1. Polysulfide der Formel (I), 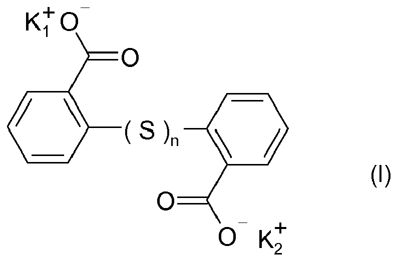 wobei die Kationen K₁⁺ und K₂⁺ unabhängig voneinander für beliebige einwertige oder für den Anteil eines beliebigen mehrwertigen Kations stehen, der einer positiven Ladung von eins entspricht, und
n für 2, 3, 4, 5 und/oder 6, vorzugsweise für 3, 4 und/oder 5 und besonders bevorzugt für 4 steht,
**dadurch gekennzeichnet, dass** eine Mischung von 10g Polysulfide der Formel (I) und 100ml Wasser nach 30 min Rückfluss bei Normaldruck und anschließendem Abkühlen auf 25°C innerhalb von 60 min einen pH > 2, bevorzugt von 2,5 - 8, besonders bevorzugt von 3 - 7, ganz besonders bevorzugt von 3,4 - 6,2 aufweist.

2. Polysulfide gemäß Anspruch 1, **dadurch gekennzeichnet, dass** es sich um eine Mischung aus zwei oder mehr Polysulfiden der Formel (I) handelt, wobei die Summe der Verbindungen mit n = 3, n = 4, n = 5 und n = 6 bezogen auf die Gesamtmenge an Polysulfidverbindungen der Formel (I) mindestens 80 %, vorzugsweise mindestens 90 % besonders bevorzugt mindestens 95% und ganz besonders bevorzugt mindestens 98% beträgt.

3. Polysulfide gemäß Anspruch 1 oder 2, **dadurch gekennzeichnet, dass** die Kationen K₁⁺ und K₂⁺ unabhängig voneinander für H⁺, ein Alkalimetallkation, insbesondere Li⁺, Na⁺, K⁺, ½ Erdalkalimetallkation, insbesondere ½ Mg²⁺, ½ Ca²⁺, für ⅓Al³⁺, für den Anteil eines Seltenerdmetallkations, der einer positiven Ladung von eins entspricht, oder für½ Zn²⁺ und ganz besonders bevorzugt für H⁺ oder für ½ Zn²⁺, insbesondere für ½ Zn²⁺ stehen.

4. Polysulfide gemäß einem der Ansprüche 1 bis 3, **dadurch gekennzeichnet, dass** eine Mischung von 10g Polysulfiden in 100ml Wasser nach 30min Rückfluss und anschließendem Abkühlen auf 25°C innerhalb von 60 min eine Leitfähigkeit < 5 mS/cm, bevorzugt < 1 mS/cm aufweist.

5. Polysulfide gemäß einem der Ansprüche 1 bis 4, **dadurch gekennzeichnet, dass** der Anteil an Verbindungen der Formel (I) mit n = 4 bezogen auf die Gesamtmenge an Polysulfiden der Formel (I) mehr als 80 %, bevorzugt mehr als 90%, ganz besonders bevorzugt mehr als 95%, insbesondere 96 - 99% beträgt.

6. Polysulfide gemäß einem der Ansprüche 1 bis 5, **dadurch gekennzeichnet, dass** der Gesamtchlorgehalt < 1%, bevorzugt < 0,3%, besonders bevorzugt < 0,1%, und ganz besonders bevorzugt < 0,03% beträgt.

7. Verfahren zur Herstellung von Polysulfiden gemäß einem der Ansprüche 1 bis 6, **dadurch gekennzeichnet, dass** es folgende Schritte umfasst:
a) Umsetzung von 2-Mercaptobenzoesäure mit S₂Cl₂ zu Polysulfiden der Formel (I), wobei K₁⁺ und K₂⁺ für H⁺ stehen und n für 2, 3, 4, 5, oder 6, vorzugsweise für 4 steht, b1) Inkontaktbringen der erhaltenen Polysulfide der Formel I bei denen K₁⁺ und K₂⁺
für H⁺ mit Wasser oder einem Gemisch von Wasser und einem inerten organischen Medium, insbesondere in unter Reaktionsbedingungen flüssigen cyclischen und/oder acyclischen Kohlenwasserstoffen, aromatischen Kohlenwasserstoffen, aliphatischen und/oder aromatischen Halogenkohlenwasserstoffen, Ethern und/oder Estern, insbesondere Toluol und Erwärmung auf eine Temperatur > 60°C, bevorzugt auf 80°C - 120°C,
und/oder
b2) Inkontaktbringen der erhaltenen Polysulfide der Formel I bei denen K₁⁺ und K₂⁺ für H⁺ stehen mit Lösungen von Metallsalzen der Kationen K₁⁺ und K₂⁺, bei Temperaturen > 60°C, und Behandlung bei Temperaturen von 80°C - 120°C.

8. Kautschukmischung enthaltend jeweils zumindest:
- einen Kautschuk
- einen Füllstoff
- Polysulfide gemäß einem der Ansprüche 1 bis 6.

9. Kautschukmischung gemäß Anspruch 8, **dadurch gekennzeichnet, dass** als Füllstoff ein oder mehrere kieselsäurebasierende Füllstoffe eingesetzt werden, wobei der Gewichtsanteil an kieselsäurebasierenden Füllstoffen mindestens 10 %, vorzugsweise mindestens 20 %, besonders bevorzugt mindestens 50 % und am meisten bevorzugt mindestens 80 % des Gesamtfüllstoffgehalts beträgt und die Kautschukmischung zusätzlich ein schwefelhaltiges Alkoxysilan enthält.

10. Kautschukmischung gemäß einem der Ansprüche 8 oder 9, **dadurch gekennzeichnet, dass** die Kautschukmischung zusätzlich einen oder mehrere Vernetzer enthält.

11. Kautschukmischung gemäß einem der Ansprüche 8 bis 10 **dadurch gekennzeichnet, dass** die Gesamtmenge an Polysulfiden gemäß einem der Ansprüche 1 bis 6 0,1 bis 15 phr, besonders bevorzugt 0,3 bis 7 phr, ganz besonders bevorzugt 0,5 bis 3 phr und am meisten bevorzugt 0,7 bis 1,5 phr beträgt.

12. Kautschukmischung gemäß einem der Ansprüche 8 bis 11, **dadurch gekennzeichnet, dass** sie als Kautschuk mindestens einen SBR-Kautschuk und mindestens einen BR-Kautschuk, vorzugsweise im Gewichtsverhältnis SBR-Kautschuk:BR-Kautschuk von 60:40 bis 90:10 enthält.

13. Kautschukmischung gemäß Anspruch 12, **dadurch gekennzeichnet, dass** sie zusätzlich mindestens einen NR-Kautschuk enthält, vorzugsweise in einem Gewichtsverhältnis SBR-Kautschuk zu BR-Kautschuk zu NR-Kautschuk von 60 bis 85 : 10 bis 35 : 5 bis 20.

14. Verfahren zur Herstellung von Kautschukmischungen gemäß einem der Ansprüche 8 bis 13 durch Mischen von mindestens jeweils einem Kautschuk, einem ggf. kieselsäurebasierenden Füllstoff, ggf. einem schwefelhaltigen Alkoxysilan und Polysulfiden gemäß einem der Ansprüche 1 bis 6, vorzugsweise durch ein mehrstufiges Mischverfahren, in welchem die Zugabe der Polysulfide gemäß einem der Ansprüche 1 bis 6 in der ersten Stufe des Mischprozesses und die Zugabe eines oder mehrerer Vernetzer in einer späteren Mischstufe erfolgt.

15. Verfahren zur Herstellung von Vulkanisaten **dadurch gekennzeichnet, dass** man die Kautschukmischung gemäß einem der Ansprüche 8 - 13 vulkanisiert, vorzugsweise bei Massetemperaturen von 100 bis 200 °C, besonders bevorzugt bei Massetemperaturen von 130 bis 180 °C.

16. Vulkanisate, insbesondere Reifen und Reifenbauteile, erhältlich durch Vulkanisation der Kautschukmischungen gemäß einem der Ansprüche 8 - 13.

17. Verwendung von Polysulfiden gemäß einem der Ansprüche 1 bis 6 zur Herstellung von Kautschukmischungen und deren Vulkanisaten.

18. Mischung enthaltend mindestens ein schwefelhaltiges Alkoxysilan, insbesondere Bis-(triethoxysilylpropyl)tetrasulfan, Bis-(triethoxysilylpropyl)disulfan, 3-(Triethoxysilyl)-1-propanthiol, polyetherfunktionalisiertes Mercaptosilan oder thioesterfunktionalisiertes Alkoxysilan und Polysulfid(e) gemäß einem der Ansprüche 1 bis 6.

19. Verwendung von schwelfelhaltigen Alkoxysilanen, insbesondere Bis-(triethoxysilylpropyl)tetrasulfan, Bis-(triethoxysilylpropyl)disulfan, 3-(Triethoxysilyl)-1-propanthiol, polyetherfunktionalisierten Mercaptosilanen oder thioesterfunktionalisierten Alkoxysilanen und Polysulfiden gemäß einem der Ansprüche 1 bis 6 zur Herstellung von Mischungen gemäß Anspruch 18.
